# EUROPEAN PATENT APPLICATION

(11) **EP 3 153 140 A1**
(43) Date of publication of application: **12.04.2017**
(21) Application number: 16193160.5
(22) Date of filing: 10.10.2016
(51) Int. Cl.: A61F 9/02

(54) **PROTECTIVE MASK FOR THE EYES, FOR SPORTS USE, COMPRISING A REMOVABLE OPTICAL SYSTEM**

(30) Priority: 09.10.2015 IT UB20154254
(71) Applicant: Anomaly Action Sports S.r.l., 30172 Mestre (Venezia) (IT)
(72) Inventor: SALMINI, Carlo, 30172 Mestre (Venezia) (IT); SALMINI, Elena, 30172 Mestre (Venezia) (IT); LIGETY, Theodore Sharp, 30172 Mestre (Venezia) (IT); SGUOTTI, Giovanni, 30172 Mestre (Venezia) (IT)
(74) Representative: Feltrinelli, Secondo Andrea

(57) **Abstract**

A protective mask for the eyes for sporting use, comprising a frame (2) having at least one internal surface (7) delimiting at least one opening (3), an optical system, (4) configured as a lens, associable to the frame (2) for closing the opening (3), and equipped with at least one first wall (11) and at least one second wall (12) spaced apart from one another and reciprocally connected, wherein the frame (2) comprises retaining members (8, 9, 10) configured to engage and internally retain, by means of shape coupling, at least one peripheral portion of the at least one first wall (11) and of the at least one second wall (12) of the optical system (4).

## Description

### TECHNICAL FIELD OF THE INVENTION

The present invention relates to a protective mask for the eyes, for sporting use, such as, for example, a ski, snowboard, mountain bike, motocross or similar mask. In particular, the present invention relates to a protective mask for the eyes comprising an optical system that can be removably connected to the mask, and to a method for connecting/removing such an optical system to/from a protective mask for the eyes.

### STATE OF THE ART

In order to protect a user's eyes while allowing good visibility during the performance of a sporting activity, the use of a protective mask or goggles for the eyes, is known.

With reference, for example, to sporting activities such as skiing, snowboarding, mountain biking, motocross or similar activities, the use of a protective mask for the eyes comprising a substantially annular frame delimiting an opening, and at least one transparent or semi-transparent lens to close this opening, is envisaged. In addition to acting as a protective barrier, the protective mask for the eyes can be configured to protect a user from glare, reflections or from exposure to excessive light. To this end, the protective mask can comprise lenses capable of adequately filtering visible radiation, for example depending on the atmospheric conditions during which the sporting activity is performed.

There are known protective masks comprising a frame to which the lens is removably connected so as to facilitate the replacement thereof with another lens with different characteristics.

According to a first embodiment, the frame comprises an internal portion that delimits the opening of the mask, along which a single peripheral groove is obtained, which serves as a seat for receiving and engaging the peripheral portion of a lens.

Such a system for connecting the lens to the frame is structurally simple but not free of drawbacks, such as, for example, the ability to firmly retain the lens in position and prevent accidental detachment thereof following impact or stress to the protective mask, or even the precision required for inserting the lens inside the peripheral groove, which can in fact make replacing the lens itself difficult.

In addition, during connection, the insertion of the lens into the frame must be forced, with the risk of ruining or deforming the lens, thus consequently distorting vision through the same.

According to a further embodiment, there are provided magnetic means associated to the frame and to the lens, capable of exerting a force of attraction such as to keep the lens connected to the frame. To remove the lens it is sufficient to push it away from the frame by applying a stress capable of exceeding the attraction exerted by the magnetic means.

This configuration is easy to use but has as drawback an increase in production costs as well as an overall increase in the weight of the mask. In addition, the aforementioned connection system is not capable of preventing any accidental detachment of the lens from the frame.

With the aim of ensuring a firm connection of a removable lens to a protective mask for the eyes, there have been developed articulated-arm mechanisms, operatively associated with the frame, configured to constrain the lens to the frame.

With respect to the previously described solutions, these systems allow a firm connection of the lens to the frame and prevent accidental detachment, even in the event of the mask being subjected to high stresses.

The aforementioned articulated-arm mechanisms have a large number of components and, consequently, a relative structural and construction complexity. In addition, the aforementioned mechanisms increase the overall weight of the protective mask and entail a greater complexity in the lens replacement procedure. There is a need in the field for a protective mask for the eyes capable of overcoming the above-specified drawbacks in the context of an easy-to-use solution.

Nevertheless, there is a need to have a protective mask for the eyes that prevents accidental detachment of the lens from the frame, in the context of an easy-to-implement solution.

### OBJECTS OF THE INVENTION

The main object of the present invention is to improve the prior art relating to a protective mask for the eyes, preferably for sporting use, comprising a removable optical system.

Another object of the present invention is to provide a protective mask for the eyes comprising a frame configured to facilitate the quick replacement of the optical system of the protective mask itself.

A further object of the present invention is to provide a protective mask for the eyes comprising a frame configured to ensure a firm and stable connection of a removable optical system, in the context of solution that is simple to implement. According to one aspect of the present invention, there is provided a protective mask for the eyes, for sporting use, according to claim 1.

According to a further aspect of the present invention, a method is also provided for connecting an optical system to such a protective mask for the eyes, for sporting use, according to claim 11.

The dependent claims relate to preferred and advantageous embodiments of the invention.

### BRIEF DESCRIPTION OF THE DRAWINGS

Further features and advantages of the present invention will become more apparent from the detailed description of a preferred, non-exclusive, embodiment of a protective mask for the eyes, for sporting use, illustrated by way of non-limiting example, in the accompanying drawings, in which:
figure 1 is a front perspective view of a protective mask for the eyes according to the present invention;
figure 2 is a rear perspective view of the protective mask for the eyes pursuant to figure 1;
figure 3 is an exploded view of a protective mask for the eyes according to the present invention;
figure 4 is a front perspective view of the protective mask for the eyes pursuant to figure 1;
figure 5 is a side cross-sectional view, along the V-V plane of the protective mask for the eyes pursuant to figure 4;
figure 6 is an enlarged detail of a cross-sectional view, along the VI-VI plane of a protective mask for the eyes pursuant to figure 4;
figure 7 is a cross-sectional view of a component associable to a protective mask for the eyes according to the present invention;

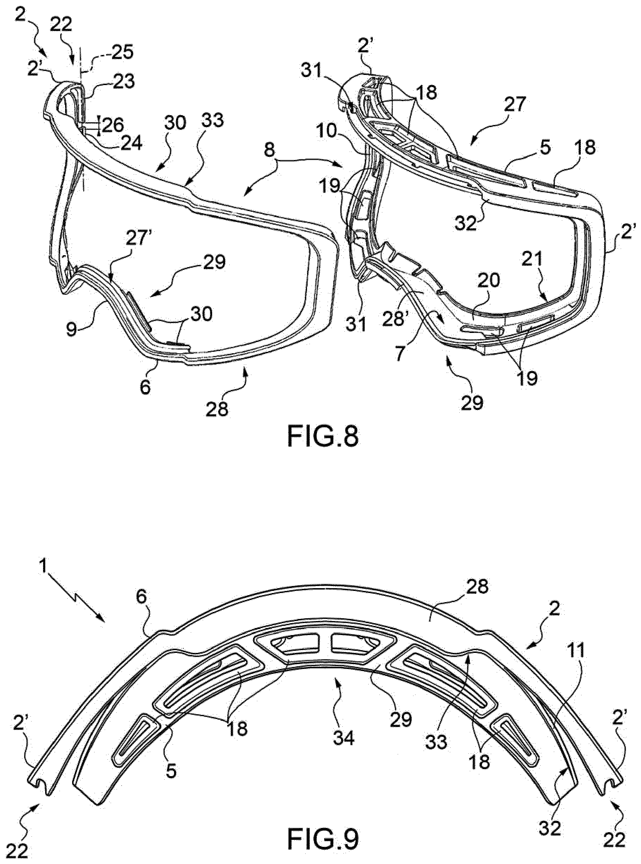

figure 8 is an exploded perspective view of a further configuration of a protective mask for the eyes according to the present invention;
figure 9 is a top view of the protective mask for the eyes pursuant to figure 8;
figures 10-12 illustrate some of the assembly steps of an optical system to a protective mask for the eyes according to the present invention.

### EMBODIMENTS OF THE INVENTION

With reference to the accompanying drawings, a protective mask for the eyes according to the present invention is generally indicated by the reference number 1.

In the description that follows, reference will be made to a protective mask for the eyes 1, for sporting activities such as skiing, snowboarding, mountain biking, motocross or similar activities, while intending that the same can nevertheless find application in other sporting and non-sporting activities.

The protective mask for the eyes 1, hereinafter simply referred to as protective mask 1, comprises a frame 2, with a substantially annular shape, if desired, that delimits at least one opening 3.

The protective mask 1 also comprises an optical system 4, in turn comprising at least one lens for closing the at least one opening 3.

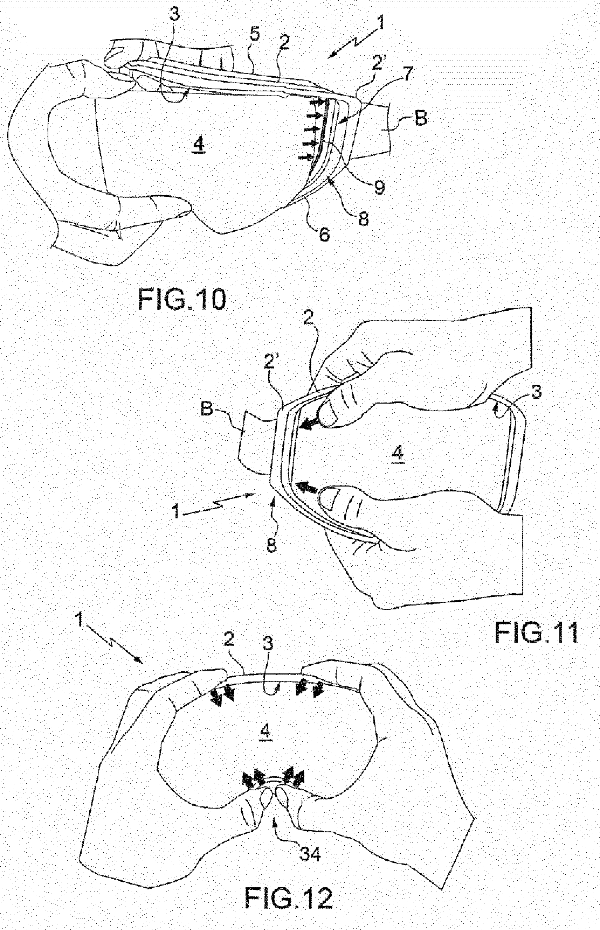

A band B or similar provided with two end portions opposite to each other is associable to opposite side ends 2' of the frame 2 to allow a user to wear the protective mask 1 around the head and to keep it firmly in position (figures 10 and 11).

The frame 2 has a rear portion 5, in use, a front portion 6, in use, spaced apart from one another and reciprocally connected by means of at least one internal surface 7.

The at least one internal surface 7 delimits the at least one opening 3.

The protective mask 1, illustrated in the accompanying drawings, has a single opening 3. Alternatively, the protective mask 1 can be configured as a pair of goggles and, for this purpose, comprise two openings 3, arranged side by side, without thereby departing from the scope of protection of the present invention. As one skilled in the art will readily understand, according to this version, each of the two openings 3 is delimited by a respective internal surface 7.

The frame 2 and the optical system 4 are configured so as to be reciprocally removably connectable by the methods that will be described hereinafter.

The protective mask 1 comprises retaining means of the optical system 4 in the frame 2, generally indicated by 8.

The retaining means 8 are provided in the frame 2 and, more precisely, along the at least one internal surface 7. The retaining means 8 are configured to retain at least one section of the peripheral edge of the optical system 4, by means of a shape coupling, according to the methods described hereinafter.

For this purpose, the retaining means 8 comprise at least one first groove 9 and at least one second groove 10.

In greater detail, the at least one first groove 9 and the at least one second groove 10 are obtained separate from each other and reciprocally spaced apart along the same section of the at least one internal surface 7, to define a first and a second, channel, arranged side to side, engageable by respective edge portions of the optical system 4.

As mentioned, the optical system 4 is configured to engage the retaining means 8 and, for this purpose, has at least one first wall 11 and at least one second wall 12 spaced apart from one another and reciprocally connected by means of suitable spacing means 13.

It is observed that in the retaining means 8, at the portion engageable by the optical system 4, the separation distance between the first groove 9 and the second groove 10 corresponds to the separation distance between the at least one first wall 11 and the at least one second wall 12 of the optical system 4.

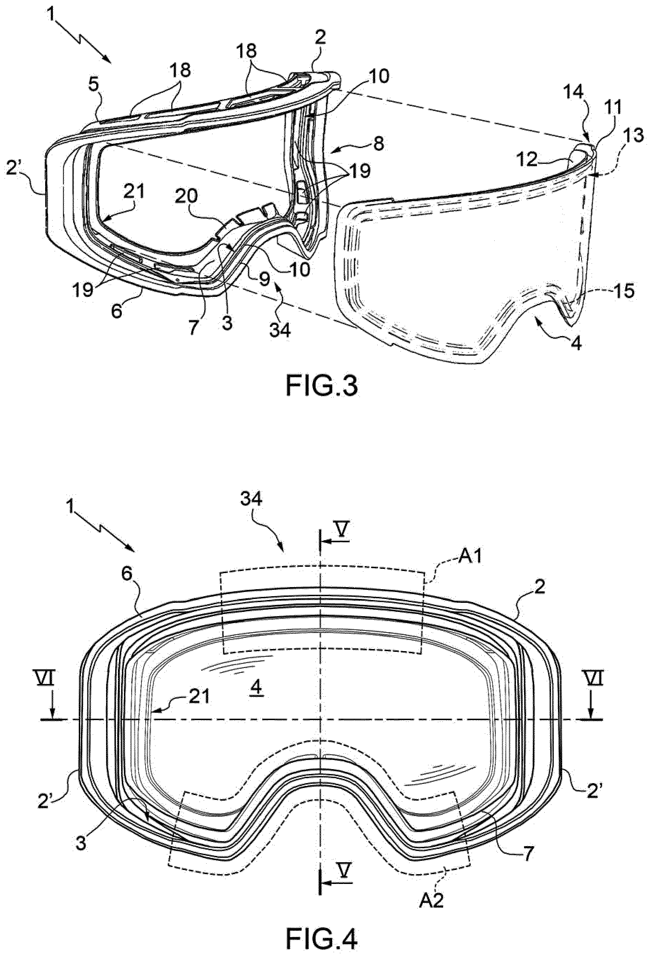

The optical system 4 can have, along at least one section of its peripheral edge, at least one gap 14 delimited between the at least one first wall 11, the at least one second wall 12 and the spacing means 13, the latter defining the bottom of the at least one gap 14 (figures 3, 5-7).

The at least one first wall 11 and the at least one second wall 12 can rise in cantilever fashion from the bottom of the at least one gap 14.

According to one version of the present invention, the length of the section of the at least one first wall 11 that rises in cantilever fashion from the bottom of the at least one gap 14, can be the same as the one of the at least one second wall 12, which rises in cantilever fashion from the at least one gap 14.

According to a further version of the present invention, the length of the section of the at least one first wall 11 that rises in cantilever fashion from the bottom of the at least one gap 14, is greater than or less than the one of the at least one second wall 12, which rises in cantilever fashion from the at least one gap 14.

It is further observed that the length of the section of the at least one first wall 11 that rises in cantilever fashion from the bottom of the at least one gap 14 can, possibly, vary along the peripheral edge of the optical system 4. Analogous considerations apply for the length of the section of the at least one second wall 12 that projects in cantilever fashion from the bottom of the at least one gap 14. In practice, the depth of the at least one gap 14 can vary along the peripheral edge of the optical system 4 itself, as a function of the specific project and use requirements

The retaining means 8 are envisaged in the frame 2 along the at least one section of the at least one internal surface 7 onto which the at least one gap 14 faces, in use. In more detail, it is observed that the at least one first groove 9 and the at least one second groove 10, in use, are respectively engaged by at least one portion of the at least one first wall 11 and by the at least one portion of the at least one second wall 12, promoting a firm connection of the optical system 4 to the frame 2 and preventing the accidental removal thereof in use.

According to one version of the present invention, not illustrated in the accompanying drawings, at least one of the at least one first groove 9 and the at least one second groove 10 can extend along the entire internal surface 7 of the frame 2.

According to this version, on observing the protective mask 1 from the front (see figure 4), the at least one first groove 9 and/or the at least one second groove 10 have, or has a substantially annular shape.

According to another version of the present invention, not illustrated in the accompanying drawings, both the at least one first groove 9 and the at least one second groove 10 extend along the entire internal surface 7, each having, analogously as described above, an annular shape.

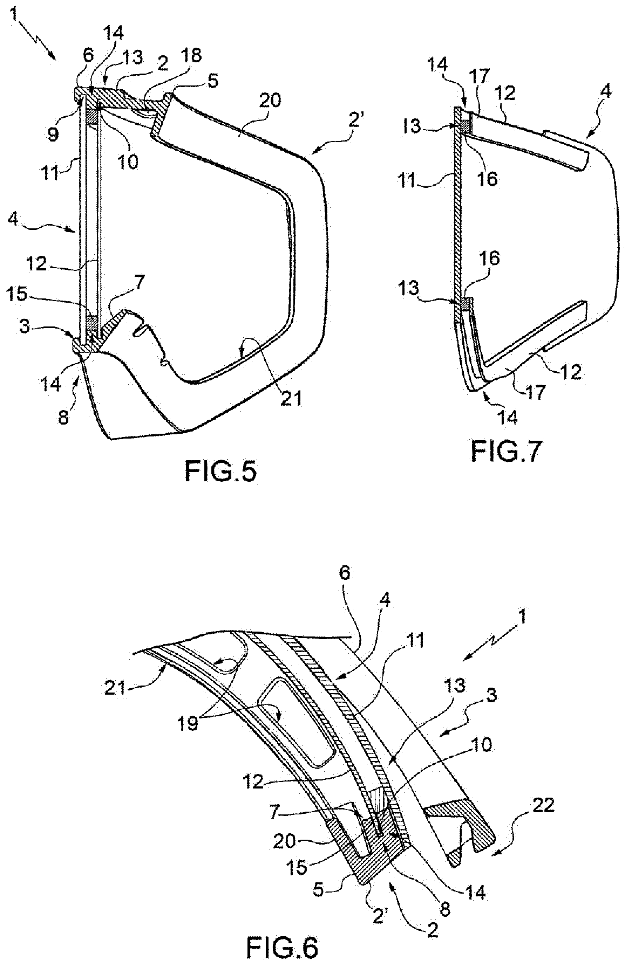

According to a further version of the present invention, the at least one first groove 9 and the at least one second groove 10 extend for at least one section of the at least one internal surface 7 in a reciprocally side-by-side position (figure 4 and 5).

With reference to what is described above, it can be deduced that the retaining means 8 can be produced along the frame 2 according to different configurations, as concerns the extension of the at least one first groove 9 and of the at least one second groove 10 and/or the reciprocal positioning thereof.

By way of a non-limiting example, figure 4 illustrates an embodiment of the protective mask 1 according to the present invention, in which the frame 2, when viewed from the front, has an upper portion and a lower portion in which the at least one first groove 9 and the at least one second groove 10 are reciprocally side-by-side. These portions are respectively delimited inside the dashed boxes A1, A2.

As mentioned, further versions of the present invention are possible, in which the at least one first groove 9 and the at least one second groove 10 are reciprocally side by side along the at least one internal surface 7 in a different way with respect to what is described above.

According to one version of the present invention, with the optical system 4 engaged in the retaining means 8, at least one section of the bottom of the at least one gap 14 can abut against the internal surface 7 (figure 5 and 6).

More precisely, at least one section of the bottom of the at least one gap 14 can abut against the portion of the internal surface 7 engaged by the optical system 4 and comprised between the at least one first groove 9 and the at least one second groove 10, thus promoting a greater stability in the connection between the optical system 4 and the frame 2.

The optical system 4 can be of the dual-lens type.

In this case, the at least one first wall 11 and the at least one second wall 12 can each be configured as a lens. The first wall 11 and the second wall 12 can be reciprocally connected, at the edge portion of the optical system 4, by spacing means 13 in the form of a seal 15.

Preferably, the seal 15 has an annular configuration such as to substantially follow the peripheral profile of the optical system 4, so as not to interfere with or restrict a user's field of vision of a user through the latter, during use of the protective mask 1.

Each lens of the optical system 4 can be made of a material such as cellulose acetate, polycarbonate, cellulose propionate, glass, polyurethane or of any material adapted to the purpose without any limitation.

According to a further version of the present invention, the optical system 4 can be of the type with a single lens to which an ancillary structure is constrained to facilitate the firm connection of the optical system 4 to the frame 2.

In accordance with this version, the at least one first wall 11 can be configured as a lens and the at least one second wall 12 can be configured as an appendage that projects from at least one first wall 11 to delimit the at least one gap 14.

In more detail, the at least one second wall 12 can have a first section 16 connected to the at least one first wall 11, in order to rise substantially at right angles therefrom, and the at least one second section 17, subsequent to the first section 16, substantially parallel to the at least one first wall 11 (see figure 7). In practice, the cross section of the second wall 12 can be substantially "L"-shaped (figure 7).

With reference to the embodiment illustrated in figure 7, the first section 16 and the second section 17 of the at least one second wall 12 are represented as separate elements from each other. It is understood that they can be alternatively produced as a single body.

The at least one second wall 12 can be constrained to the at least one first wall 11 by means of gluing or the like.

According to one version of the present invention, the at least one second wall 12 is configured as an ancillary frame constrained to the internal surface, in use, of the at least one first wall 11.

According to a further version of the present invention, the first section 16 of the at least one second wall 12 could be a seal or the like.

According to another version, the at least one second wall 12 could be made in a single piece with the at least one first wall 11, for example by means of a moulding or injection or similar process, and can be formed from the same material of the at least one first wall 11 or of a different material.

The optical system 4 is transparent or semi-transparent, to allow vision through the same.

A reflective or mirror coating or a mirror can be applied on the external surface in use of the optical system 4, as a function of the specific usage requirements

It is understood that the optical system 4 can be configured to filter and reduce the intensity of the visible radiation that passes through it, in order to prevent glare phenomena, or to reduce reflections or emphasise a particular colour range as a function of the specific usage requirements without any limitation. By way of example, it is observed that on the surface of the optical system 4, which in use is internal to the protective mask 1, at least one anti-reflection and/or hydrophobic and/or oleophobic coating can be applied as a function of the specific requirements of use without any limitation.

The optical system 4 and the frame 2, associated to each other, delimit the internal volume of the protective mask 1 itself comprised between the optical system 4 and a wearer's face.

During use, the face of a user wearing the protective mask 1 emits heat which can cause fogging of the optical system 4, thus obstructing vision through the same. In order to prevent or mitigate this phenomenon, the frame 2 can have ventilation openings adapted to promote the passage of air inside the protective mask 1.

By way of example, the protective mask 1 can have first through openings 18, produced at the upper portion of the frame 2, in such a position as not to interfere with the retaining means 8 and with the optical system 4.

Optionally, according to a further version of the present invention, the protective mask 1 can comprise further through openings 19 at the rear and/or lower and/or side portion of the frame 2 to integrate the first through openings 18, to promote a flow of air into the internal volume of the protective mask 1.

A layer of a material permeable to air but not to water, not illustrated, can be applied along the frame 2, to close the first through openings 18 and the further through openings 19 in order to promote the passage of air and prevent water, snow or foreign bodies from entering the aforementioned internal volume of the protective mask during 1 use.

Such a layer of a material that is permeable to air but not to water can be made starting from water repellent materials or, possibly, treated with substances capable of making it hydrophobic and prevent the accumulation of water and/or wet snow that could obstruct the passage of air through the layer itself, thus reducing the efficiency of the ventilation inside the protective mask 1.

According to a further version, the optical system 4 can have through openings, not illustrated in the accompanying drawings, to ensure a flow of air into the internal volume of the protective mask 1, in order to prevent and mitigate fogging phenomena of the optical system 4 itself. These through openings can be equipped with at least one layer of material that is permeable to air but not to water, capable of promoting the passage of air and preventing foreign bodies from entering the internal volume of the protective mask 1 during use. The optical system 4 itself can comprise further ventilation systems, possibly integrated into the seal 15 when envisaged.

The frame 2 can be made of rigid or semi-rigid material so as to be able to firmly retain the optical system 4, having a limited deformability.

For example, the frame 2 can be made from a material such as ethylene vinyl acetate (EVA), polyurethane, nylon, rubber, etc.

The frame 2 can have a differentiated rigidity. For example, at least the portion of the frame 2 to which the optical system 4 is connectable can have a greater rigidity than that of the other portions of the frame 2 itself.

For this purpose, the frame 2 can be produced by means of a moulding process that envisages the use of at least one first material and one second material, such as for example an injection or bi-injection or over-moulding or co-injection or similar process.

The at least one first material can have different mechanical resistance properties than those of the at least one second material.

In particular, the at least one first material can have a greater rigidity than that of the at least one second material, in order to achieve the above-indicated objects. According to one version of the present invention, not illustrated in the accompanying drawings, the frame 2 can internally comprise at least one reinforcing member or core, if desired, completely or at least partly embedded in the frame 2 itself.

The at least one core can be positioned inside the frame 2, in order to reinforce, at least locally, the frame 2 itself, according to specific requirements of use.

The core can be made starting from a material characterised by a high rigidity and by a low specific weight, comparable at least to the same order of magnitude as that of the material constituting the frame 2, in order not to negatively affect the overall weight of the protective mask 1, and therefore the inertia thereof. According to one aspect of the present invention, in order to facilitate the connection between the optical system 4 and the frame 2, and to reduce the time required to replace the optical system 4 itself, at least one first groove 9 and the at least one second groove 10 can have a reduced depth. In this case, the at least one gap 14 in turn has a reduced depth.

In general, the depth of the at least one first groove 9 and of the at least one second groove 10 is sized according to the specific characteristics of the project, such as for example the shape of the protective mask 1, the materials used, the intended use for the protective mask 1 itself, etc.

It is however observed that the retaining means 8, albeit in the presence of at least one first groove 9 and/or of at least one second groove 10 having a reduced depth, guarantee a firm and stable engagement of the optical system 4, in that they retain both walls 11, 12 thereof.

The protective mask 1 can possibly comprise, at the rear portion 5, an edge or flexible eyelid 20 adapted to abut against the user's face, in use.

The flexible edge 20 delimits at least one second opening 21, substantially aligned with the at least one first opening 3.

The flexible edge 20 is deformable so as to be able to adapt to the shape of a wearer's face against which it rests, in use.

According to one version of the present invention, at least one layer of soft and deformable material having an annular shape, such as a foam, not illustrated in the accompanying drawings, can be applied on the flexible edge 20, in order to give a greater sensation of comfort to a user wearing the protective mask 1.

According to one aspect of the present invention, the protective mask 1 can comprise connecting means for a band B adapted to retain, in use, the protective mask 1 in position on the head of a user, generally indicated by 22.

The connecting means 22 are envisaged in proximity to the opposite side portions 2' of frame 2.

Each side portion 2' of the frame 2 therefore has respective connecting means 22. According to one version of the present invention, the connecting means 22 are configured to allow the removable connection of the band B to the protective mask 1.

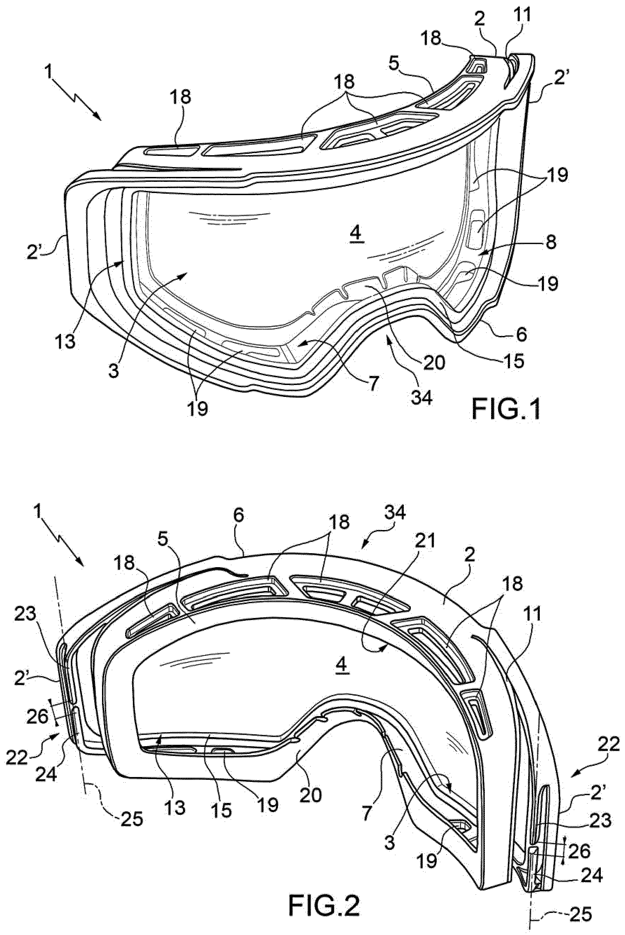

For this purpose, the connecting means 22 are configured to allow the engagement and retention in position of end portions of the aforementioned band B, not illustrated in the accompanying drawings, configured as a hollow tubular element. With reference to the embodiment illustrated in figure 2, the connecting means 22 are shaped as a first arm 23 and a second arm 24, each equipped with a respective free end, which extend from opposite portions of the frame 2. The first arm 23 and the second arm 24 are substantially aligned along a same axis 25 until they come in proximity one to the other at the respective free ends.

The free ends of the first arm 23 and of the second arm 24 are spaced apart from one another to define a passage 26 through which to introduce the tubular element of a respective end portion of the band B.

It is also observed that the first arm 23 and the second arm 24 are spaced apart from the frame 2 to define a space or slot for the passage of a portion of band B itself.

The first arm 23 and the second arm 24 have a different length from each other to facilitate the insertion/removal of the end portion of band B into/from the connecting means 22 as better clarified hereinafter. According to a preferred version of the present invention, the first arm 23 has a length greater than that of the second arm 24.

To connect the band B to the protective mask 1, one proceeds by introducing the end portion of the band B, configured as a tubular element, through the passage 26 present between the first arm 23 and the second arm 24, and therefore, by fitting the tubular element along the first arm 23.

With the tubular element almost completely fitted along the first arm 23, one then proceeds by fitting the opposite end of the tubular element along the second arm 24, until the tubular element itself is fully engaged along the first arm 23 and the second arm 24.

To disengage the tubular element from the connecting means 22, one proceeds in an inverse manner with respect to what has been previously described. According to a further version of the present invention, illustrated in the accompanying drawings 8 and 9, the frame 2 can be formed by at least two parts 27, 28 that are reciprocally connectable to each other.

In greater detail, the frame 2 can comprise a rear frame 27 and a front rim 28 that are reciprocally connectable to delimit the at least one opening 3.

In this case, the at least one internal surface 7 of the frame 2 can comprise a first portion 27', corresponding to the internal surface of the rear frame 27 and a second portion 28', corresponding to the internal surface of the front rim 28. According to one version of the present invention, the rear frame 27 and the front rim 28 can be permanently reciprocally connected by means of gluing or a similar method adapted to determine a permanent constraint.

According to a further version of the present invention, the frame 2 can comprise interlocking means, generally indicated by 29, for the removable connection of the rear frame 27 to the front rim 28.

The interlocking means 29 comprise engaging members 30 and respective supplementary receiving seats 31. The engaging members 30 are configured to be received in the respective seats 31 by means of a shape coupling, preferably interlocking or, in general, by means of a coupling with interference, to ensure a firm connection between the rear frame 27 and the front rim 28 and prevent an accidental detachment thereof.

According to one version of the present invention, the engaging members 30 can posteriorly project from the front rim 28 and engage in respective seats 31 produced in the rear frame 27. In accordance with this version, the seats 31 are envisaged at a front surface 32, in use, of the rear frame 27.

By way of a non-limiting example, the engaging members 30 can be configured as hooks, pins or the like without any limitation.

According to a further version of the present invention, not illustrated in the accompanying drawings, the engaging members 30 can frontally project from the rear frame 27 and engage in respective seats 31 produced in the front rim 28. In accordance with this version, the seats 31 are envisaged at a rear surface 33, in use, of the front rim 28.

Further versions are nevertheless possible, wherein the engaging members 30 project from both the rear frame 27 and from the front rim 28 and engage in respective supplementary seats 31 in turn envisaged in the front rim 28 and in the rear frame 27.

The frame 2 comprising two parts 27, 28 that are removably associable with each other, achieves the same advantages as set forth in relation to the previous version, produced in a single body, as concerns the ease and rapidity of replacement of the optical system 4, and the firmness of the connection between the optical system 4 and the frame 2.

Additionally, if the front rim 28, which among other things serves as a protecting member for the rear frame 27, should be damaged, it is possible to proceed with the replacement thereof with a new front rim 28, thus ensuring the integrity and the efficiency of the protective mask 1 without requiring the full replacement of the frame 2.

As mentioned, the frame 2 can comprise portions made of materials having different rigidity. In this regard, it is observed that the rear frame 27 can be made of a different material than the material of the front rim 28 and can be less rigid. By way of a non-limiting example, the rear frame 27 can be made starting from a material such as a yielding plastic, for example TPU, EVA, Nylon, rubber or the like, to promote at least an elastic deformability thereof.

The front rim 28 can be made starting from a material having a greater rigidity than that of the rear frame 27 such as, for example, TPU, EVA, Nylon, rubber or a composite material such as CFRP, GRP, wood or the like.

As regards the configuration of the retaining means 8 in a frame 2 comprising a rear frame 27 and a front rim 28, which are associable with each other, analogous considerations to those previously described apply.

According to a further version of the present invention at least one of the rear frame 27 and the front rim 28 can comprise at least one of the at least one first groove 9 and the at least one second groove 10, and the other of the rear frame 27 and the front rim 28 can comprise at least the other between the at least one second groove 10 and the at least one first groove 9.

According to another version of the present invention, the rear frame 27 and the front rim 28 can be shaped in such a way that by reciprocally associating them one can configure at least one section of the at least one first groove 9 or of the at least one second groove 10 at the abutment zone between the front surface 31 of the rear frame 27 and the rear surface 33 of the front rim 28.

A method for connecting an optical system 4 to a frame 2 of a protective mask 1 is described below.

A protective mask 1, devoid of an optical system 4, and comprising a frame 2 equipped with retaining means 8, is initially provided.

The retaining means 8 are of the type comprising at least one first groove 9 and at least one second groove 10, which each extend along the at least one section of the at least one internal surface 7 of the frame 2.

There is therefore provided an optical system 4, for example a dual-lens system, comprising at least one first wall 11 and at least one second part 12 reciprocally connected to each other to define at least one gap 14, which extends in proximity to at least one section of the peripheral edge of the optical system 4 itself.

The optical system 4 is subsequently brought closer to the frame 2, aligning it with the at least one opening 3. One then proceeds by engaging at least one portion of the edge of the optical system 4 in the retaining means 8.

The step of engaging at least one portion of the optical system 4 in the retaining means 8 envisages the introduction of at least one section of the edge portion of the at least one first wall 11 into at least one first groove 9 and the introduction of at least one section of the edge portion of the at least one second wall 12 into the at least one second groove 10.

One proceeds in an analogous manner until the optical system 4 is fully engaged in the retaining means 8.

One subsequently proceeds by laterally compressing the frame 2 against the optical system 4, which is engaged by the retaining means 8, to ensure the correct shape coupling of the edge portions of the at least one first wall 11 and of the at least one second wall 12 respectively inside the at least one first groove 9 and the at least one second groove 10.

In detail, it is observed that the side compression of the frame 2 against the optical system 4 is performed at least at a central median portion 34 of the frame 2.

To remove the optical system 4 of the frame 2, one proceeds in a substantially inverse manner with respect to what is set out above.

Initially, one proceeds by widening the frame 2 at least at its central median portion 34, namely by reciprocally separating the upper portion and the lower portion of the frame 2 itself.

The optical system 4 is thereby at least partially released from the retaining means 8.

Subsequently, one proceeds by forcing the optical system 4 away from the frame 2, in order to extract the edge portions of the first wall 11 and of the second wall 12 that are still engaged in the retaining means 8 until complete separation of the optical system 4 from the frame 2.

If the frame 2 comprises a rear frame 27 and a front rim 28 that are reciprocally connectable, the rear frame 27 and the front rim 28 must be connected to each other before the optical system 4 is connected to the frame 2.

For this purpose, one proceeds by introducing the engaging members 30 that project from one or other of the rear frame 27 and the front rim 28, inside respective supplementary seats 30 produced in the other between the front rim 28 and the rear frame 27.

One skilled in the art will readily understand that with a protective mask for the eyes 1 according to the present invention, it is possible to easily replace the optical system 4 more quickly since the precision typical of the systems of the prior art is not therefore required in the context of a simple-to-implement solution. The protective mask for the eyes 1 according to the present invention allows the drawbacks of the known solutions to be overcome, in that the retaining means 8 ensure a firm and stable connection of the optical system 4 to the frame 2 albeit having an extremely reduced number of components, in favour of a greater ease of use and of a reduction in the weight of the protective mask itself.

The absence of articulated mechanisms to activate, or of parts to connect to the protective mask to constrain the optical system 4 to the frame 2, facilitates replacement of the optical system 4 in any condition of use.

Not only, the retaining means 8 according to the present invention, while presenting an extremely simple configuration, prevent accidental detachment of the optical system 4 from the frame 2, in that they firmly retain both the walls 11, 12 thereof inside the frame 2.

As indicated above, at the end of the connecting step of an optical system 4 to the frame 2, the latter is clamped against the end portions of the at least one first wall 11 and of the at least one second wall 12, firmly engaging them in the retaining means 8. Indeed, the frame 2 acts as a clamp, clamping the optical system 4 therein, thus preventing an accidental detachment thereof.

To allow the removal of the optical system 4 from the frame 2, it is in fact necessary to preliminarily proceed with the widening of the central median portion 34 of the frame 2 itself, thus at least partially disengaging the optical system 4 from the retaining means 8. Alternatively, it is not possible to remove the optical system 4 from the protective mask 1 without causing damage to the optical system 4 itself.

The protective mask for the eyes 1 descripted above is susceptible to a number of modifications and variants within the scope of the scope of protection of the claims set forth below.

## Claims

1. A protective mask for the eyes, for sporting use, comprising a frame (2) delimiting at least one opening (3), said frame (2) having at least one internal surface (7) for delimiting said at least one opening (3) and comprised between a rear portion (5) and a front portion (6) spaced apart from one another,
at least one optical system (4), configured as a lens, that can be associated with said front portion (6) for closing said at least one opening (3), said optical system (4) having at least one first wall (11) and at least one second wall (12) spaced apart from each other and reciprocally connected to define at least one gap (14) extending along at least one section of the perimeter edge of said at least one optical system (4),
retaining means (8) integral with said frame (2) for the removable connection of said at least one optical system (4) to said frame (2), said retaining means (8) being provided along at least one section of said inner surface (7) on which, in use, at least one said gap (14) faces,
**characterized in that** said retaining means (8) are configured to engage inside of specific seats, by means of shape coupling, at least one section of the perimeter portion of said at least first wall (11) and at least one section of the perimeter portion of said at least second wall (12), to ensure the firm connection of said at least one optical system (4) to said frame (2).

2. A protective mask for the eyes according to claim 1, wherein said retaining means (8) comprise at least one first groove (9) and at least one second groove (10) separated and reciprocally spaced to define at least one first and at least one second channel or seat for the retention respectively of at least one portion of said at least one first wall (11) and at least one portion of said at least one second wall (12).

3. A protective mask for the eyes according to claim 2, wherein said at least one first groove (9) and said at least one second groove (10) extend adjacent to one another along at least one section of said inner surface (7).

4. A protective mask for the eyes according to any one of the preceding claims, wherein said optical system (4) is of the type with double lens, wherein said at least one first wall (11) and said at least one second wall (12) are each configured as a lens and are connected to each other, at the perimeter of said optical system (4), by means of substantially annular spacing means (13), wherein said spacing means (13) define the bottom of said at least one gap (14).

5. A protective mask for the eyes according to any one of claims 1 to 3, wherein said at least one first wall (11) is configured as a lens and said at least one second wall (12) is configured as an appendix which protrudes from said at least one first wall (11) to define said at least one gap (14), said at least one second wall (12) having a first section (16) substantially orthogonal to said at least one first wall (11) and a second section (17) subsequent to said first section (16) and substantially parallel to said at least one first wall (11).

6. A protective mask for the eyes according to any one of the preceding claims, wherein said frame (2) comprises a rear frame (27), a front rim (28) and interlocking means (29) for the removable connection between said rear frame (27) and said front rim (28).

7. A protective mask for the eyes according to any one of the preceding claims, wherein at least one between said rear frame (27) and said front rim (28) comprises at least one of said at least one first groove (9) and said at least one second groove (10) and/or the other between said rear frame (27) and said front rim (28) comprises at least the other between said at least one second groove (10) and said at least one first groove (9).

8. A protective mask for the eyes according to any one of claims 6 or 7, wherein at least one section of said at least one first groove (9) or of said at least one second groove (10) is defined by said rear frame (27) and said front rim (28) when associated with each other.

9. A protective mask for the eyes according to any one of the preceding claims, wherein said frame (2) has portions with different rigidity with respect to each other, being formed from at least one first material and at least one second material, wherein said at least one first material has different mechanical resistance properties from those of said at least one second material.

10. A protective mask for the eyes according to any one of the preceding claims, wherein when said optical system (4) is connected to said frame (2) and engaged in said retaining means (8), at least one bottom portion of said at least one gap (14) is in abutment against a section of said inner surface (7) comprised between said at least one first groove (9) and said at least one second groove (10).

11. Method for the assembly of a protective mask for the eyes, for sporting use, comprising the steps of:
providing a frame (2) delimiting at least one opening (3), wherein said frame (2) has a rear portion (5), in use, a front portion (6), in use, spaced apart from one another, and at least one internal surface (7) interposed between said rear portion (5) and said front portion (6), said at least one internal surface (7) delimiting said at least one opening (3);
providing an optical system (4), configured as a lens, comprising at least one first wall (11) and at least one second wall (12) spaced apart from each other and reciprocally connected to define at least one gap (14) extending in correspondence with at least one section of the perimeter edge of said at least one optical system (4),
connecting said optical system (4) to said frame (2) by retaining means (8),
**characterized in that** said connecting step comprises
engaging, by means of shape coupling, at least one perimeter portion or the entire perimeter portion of said at least one first wall (11) and at least one perimeter portion or the entire perimeter portion of said at least one second wall (12) in said retaining means (8) until said optical system is firmly engaged (4) on said frame (2).

12. Method for the assembly of a protective mask for the eyes according to claim 11, wherein said engaging comprises the phase of introducing at least one section of said perimeter portion or the entire perimeter portion of said at least one first wall (11) in a first groove (9) provided in said retaining means (8) and of introducing at least one section of said perimeter portion or the entire perimeter portion of said at least one second wall (12) in at least one second groove (10) provided in said retaining means (8).

13. Method for the assembly of a protective mask for the eyes according to any one of claims 11 or 12, comprising the step of laterally compressing said frame (2) clamping it against said optical system (4), when said optical system (4) is engaged in said retaining means (8), acting at least at a central median portion (34) in said frame (2) in order to firmly engage said at least one first wall (11) and said at least one second wall (12) in said retaining means (8).

14. Method for the removal of an optical system (4) from a protective mask for the eyes embodied according to any one of claims 1 to 10 comprising the phase of grasping said frame (2) at opposite sides of its central median portion (34), expanding said frame (2) thus releasing at least one perimeter portion of said optical system (4) from said at least one first groove (9) and/or from said at least one second groove (10) of said retaining means (8), pushing said optical system (4) away from said frame (2) up to completely release said optical system (4) from said retaining means (8).
